**Europäisches Patentamt**

**European Patent Office**

**Office européen des brevets**

(11) Publication number : **0 475 926 A2**

# EUROPEAN PATENT APPLICATION

(21) Application number : **91870141.8**

(22) Date of filing : **09.09.91**

(51) Int. Cl.$^5$ : **C07C 51/265,** C07C 63/333

(30) Priority : **10.09.90 US 579504**

(43) Date of publication of application :
**18.03.92 Bulletin 92/12**

(84) Designated Contracting States :
**AT BE CH DE DK ES FR GB GR IT LI LU NL SE**

(71) Applicant : **MONSANTO COMPANY**
**800 North Lindbergh Boulevard**
**St. Louis Missouri 63167 (US)**

(72) Inventor : **Schaefer, George Francis**
**9614 Huron**
**Olivette, Missouri 63132 (US)**

(74) Representative : **Ernst, Hubert**
**Monsanto Services International S.A., Patent**
**Department, Avenue de Tervuren 270-272,**
**Letter Box No. 21**
**B-1150 Brussels (BE)**

(54) **Oxidation of dialkyl polyaromatics to dicarboxypolyaromatics.**

(57)  A process for the production of poly-aromatic acids is disclosed in which the alkyl substituents of an alkyl substituted poly-aromatic compound are oxidized with an oxygen containing gas in the presence of a catalytic amount of a halogen-free metal catalyst composition, the oxidation being carried out in an organic aliphatic carboxylic acid solvent having from 2 to about 7 carbon atoms.

EP 0 475 926 A2

Jouve, 18, rue Saint-Denis, 75001 PARIS

## BACKGROUND OF THE INVENTION

This invention relates to the production of poly-carboxyl poly-aromatic acids, for use in the high performance plastics industry, by catalytic oxidation of poly-alkyl poly-aromatic compounds using molecular oxygen and to a novel, halogen-free catalyst for the oxidation process.

## DESCRIPTION OF THE PRIOR ART

It is known, in U.S. Patent 3,115,520, that aromatic hydrocarbons having at least one, and preferably two or more, oxidizable substituents may be converted into carboxylic acid products by contact with an oxidizing gas under carefully controlled conditions in the presence of a suitable solvent such as a mono-basic aliphatic acid reaction medium having 2 to 6 carbon atoms per molecule such, for example, as acetic acid, and also in the presence of known oxidation catalysts such as manganese and/or cobalt and also in the presence of a source of bromine. The preferred mixed metal catalysts of the prior art have manganese compounds in the proportions of 1 to 2 parts by weight of the manganese salt per part by weight of the other metal compound.

Auto-oxidation of alkyl-aromatic compounds to produce aromatic acids was disclosed in a series of five patents issued in the United States in 1958 to Saffer et al., U.S. Patent Nos. 2,833,816-820. These patents taught the use of catalytic amounts of a heavy metal carboxylate salt, for example, manganese carboxylates, and of bromine. In U.S. Patent 2,833,816 the disclosed catalyst contains a heavy metal or a mixture of metals selected from the group consisting of manganese, cobalt, nickel, chromium, vanadium, molybdenum, tungsten, tin and cerium, and preferably being manganese and cobalt; an aliphatic acid of 1-8 carbon atoms; and a source of bromine. The metal can be provided as a metal, as a metal complex or as a salt, with the preferred form being the salt of an aliphatic carboxylic acid. Mixed metal catalysts are exemplified by a mixture containing one part cobalt and one-to-three parts manganese. The bromine can be provided as elemental bromine, hydrobromic acid, an ionic bromide salt, or an organic bromine-containing compound. The metal bromide salt, which may be added directly or formed during the reaction from the above sources of these components, should be present in the amount of 0.1-10% by weight, based upon the concentration of the aromatic reactant. The aromatic compounds which may be oxidized in the presence of this catalyst contain alkyl groups attached through either primary or secondary alkyl carbons.

Linear dicarboxy polyphenyls are of great use in liquid crystal polymers. For example, replacing terephthalic acid with 4,4'-dicarboxybiphenyl or 4,4''-dicarboxy-p-terphenyl imparts greater rigidity and stability to polyester polymers with a resulting improvement in performance characteristics. However, up to now such benefits have been unavailable because an environmentally safe, economical method for the preparation of the linear dicarboxypolyphenyls was lacking.

A number of methods for the preparation of 4,4'-dicarboxybiphenyl have been described in the prior art, though none of them have found commercial application. United Kingdom Patent 2,155,921 teaches carboxylation of 4-alkylbiphenyl in the presence of hydrogen fluoride and boron trifluoride (HF/BF$_3$) followed by oxidation of the alkyl group. This process has the disadvantage of requiring stoichiometric amounts of an expensive component, hydrogen fluoride (HF). Another expensive route is taught in Japanese Patent Application JP57/149,243, now issued as JP83/46,494, In this patent 4,4'-dicarboxybiphenyl is formed from the dipotassium salt of diphenic acid which can be isomerized with cadmium catalysts at high temperature in the presence of carbonic acid gas, but only with low to moderate yields. Another unsatisfactory method, taught in U.S. Patent 3,383,402, is the diacylation of biphenyl with acetyl chloride which requires stoichiometric amounts of aluminum chloride (AlCl$_3$).

Methods have also been disclosed for the production of 4,4'-dicarboxybiphenyl from halogenated aromatic compounds. U.S. Patent 3,636,082 teaches carboxylation of 4,4'-dibromobiphenyl while EP 0,206,543 teaches the coupling of p-chlorobenzoic acid. Both methods have the potential to generate halogenated biphenyls as undesirable by-products.

U.S. Patent 3,296,280 discloses the oxidation of 4-t-butyl-4'-carboxybiphenyl by nitrogen dioxide (NO$_2$) at high temperatures to produce 4,4'-dicarboxybiphenyl, but the examples show that the reaction proceeds only in low yield and produces undesirable nitrated by-products. Oxidation of 4,4'-dimethylbiphenyl by nitrogen dioxide (NO$_2$) is reported in U.S. Patent 3,631,097; but, at this time, there is no economical route to 4,4'-dimethylbiphenyl. All economical syntheses of methyl-substituted biphenyl produce a mixture of isomers with a low yield of the p,p'-isomer.

A need remains for an improved, halogen-free process for the oxidation of dialkyl polyaromatic compounds to produce para-oriented aromatic acids such as 4,4'-dicarboxybiphenyl.

## SUMMARY OF THE INVENTION

It is an object of this invention to provide a process for the production of poly-aromatic acids, such as poly-carboxyl poly-aromatic acids, from alkyl substituted poly-aromatic compounds.

It is another object of this invention to provide a catalyst composition for the efficient oxidation of the alkyl substituted poly-aromatic compounds.

It is a further object of this invention to provide a halogen-free catalyst composition for the efficient oxidation of the alkyl substituted poly-aromatic compounds.

It is yet another object of this invention to provide a process for the production of 4,4'-dicarboxybiphenyl which is also called 4,4'-biphenyl dicarboxylic acid.

These and other objects are met by this invention which is directed to a process for the production of poly-aromatic acids, such as poly carboxyl poly-aromatic acids, comprising oxidizing with oxygen the alkyl substituents of an alkyl substituted poly-aromatic compound, having at least two alkyl substituents attached to the aromatic rings, in the presence of a catalytic amount of a halogen-free metal catalyst composition comprising a source of cobalt, such as a cobalt salt, and a minor amount of a source of manganese, such as a manganese salt. The alkyl substituents may be attached to one aromatic ring or they may be attached to more than one aromatic ring. Preferably at least one alkyl substituent is attached to each terminal, or end, aromatic ring such as, for example, 4,4'-diisopropyl biphenyl, 4,4''-diisopropyl terphenyl and 2,6-diisopropyl naphthalene. The oxidizing is carried out in a solvent for the reactants and the intermediates, the solvent comprising an organic aliphatic carboxylic acid having from 2 to about 7 carbon atoms. In one of its specific aspects, this invention contemplates the oxidation of 4,4'-diisopropyl biphenyl to produce 4,4'-dicarboxy biphenyl (4,4'-biphenyl dicarboxylic acid).

## DESCRIPTION OF THE PREFERRED EMBODIMENTS

The present invention relates to a process for the production of poly-aromatic acids, such as poly-carboxyl poly-aromatic acids, which comprises oxidizing with oxygen the alkyl substituents of an alkyl substituted poly-aromatic compound, having at least two alkyl substituents attached to the aromatic rings, in a solvent for the reactants and the intermediate compounds, said solvent comprising an aliphatic carboxylic acid having from 2 to about 7 carbon atoms, in the presence of a catalytic amount of a halogen-free metal catalyst comprising a source of cobalt and a minor amount of a source of manganese. The alkyl substituents may be attached to one aromatic ring or attached to more than one aromatic ring. Preferably at least one alkyl substituent is attached to each terminal, or end, aromatic ring such as, for example, 4,4'-diisopropyl biphenyl, 4,4''-diisopropyl terphenyl and 2,6-diisopropyl naphthalene.

The starting material used in the present invention is an alkyl-substituted poly-aromatic compound having at least two alkyl substituents attached to the aromatic ring. The aromatic ring structure can be polyphenyls such as biphenyl and terphenyl, polycyclic compounds such as naphthalene or anthracene, or other alkyl-substituted aromatic compounds such as alkyl-substituted diphenyl ethers. The alkyl substituents may have any number of carbon atoms. However, it is desired, as a practical matter, to limit the alkyl substituents to alkyls having from 1 to about 20 carbon atoms and it is preferred that the alkyl substituents be alkyl groups having from 1 to about 5 carbon atoms. The alkyl substituents may be attached to the ring by a primary, secondary or tertiary carbon atom. A preferred starting material is 4,4'-di-isopropyl biphenyl; however, other suitable starting materials including, for example, 4,4'-diethylbiphenyl, 4-methyl-4'-isopropylbiphenyl, 4,4'-di-sec-butylbiphenyl, 3,4'-diisopropylbiphenyl, 3-methyl-4'-(2-pentyl)biphenyl, 3-sec-butyl-4'-ethylbiphenyl, 3,3'-diisopropylbiphenyl, 3,3'-di-(2-pentyl)biphenyl, 2,6-diisopropylnaphthalene, 2,7-diisopropylnaphthalene, 2-ethyl-6-methylnaphthalene, 2-methyl-6-isopropylnaphthalene, 4,4''-diisopropylterphenyl, 4,4''-diethylterphenyl, 4-ethyl-4''-methylterphenyl are also contemplated. Other aromatic reactants suitable for oxidation by this reaction will be apparent to those skilled in the art.

In accordance with the process of this invention, carboxyl aromatics are prepared by catalytic oxidation of alkyl radicals attached to the aromatic rings of an alkyl-substituted aromatic compound. The oxygen may be supplied as air, as pure oxygen gas, or as oxygen diluted with inert gases such as, for example, nitrogen, argon or carbon dioxide. The total amount of molecular oxygen added to the reaction system is dependent upon the aromatic compound being oxidized. The minimum amount of oxygen added to the reaction mixture is the stoichiometric amount required to react with the alkyl groups being oxidized to carboxyl (COOH) groups. The flow of oxygen through the reactor in which the oxidation takes place is generally within the range of from about 2 moles to about 500 moles of oxygen per mole of the poly-alkyl poly-aromatic reactant and is preferably within the range of from about 5 moles to about 100 moles of oxygen per mole of the reactant. The flow of oxygen through the reactor can be used to agitate the reaction mixture during the oxidation reaction; however, a mechanical agitator may also be desired.

This invention provides a novel, halogen-free metal catalyst composition as the oxidation catalyst. The catalyst composition comprises a source of cobalt such as, for example, a cobalt salt and a source of a minor amount of at least one other metal selected from the group consisting of cerium, chromium, gadolinium, manganese, molybdenum, tin, tungsten, vanadium and zirconium such as, for example, a manganese salt or a chromium salt. The cobalt and the other metal, or metals, may be provided as inorganic compounds, such as hydroxides and carbonates, and as salts of organic acids, such as acetic acid, propionic acid and benzoic acid, or of halogen-free inorganic acids, such as sulfuric acid. More specifically, the cobalt may be provided in the form of any halogen-free cobalt salt that is soluble in the reaction medium such as, for example, cobalt acetate or cobalt sulfate. Similarly, the other metal may be provided in the form of any halogen-free metal salt that is soluble in the reaction medium such as, for example, manganese acetate or manganese sulfate. The preferred catalyst composition comprises a source of cobalt such as, for example, a cobalt salt and a source of a minor amount of manganese such as, for example, a manganese salt. In the preferred catalyst composition, the molar ratio of cobalt to manganese is generally in the range of from about 10 moles of cobalt per mole of manganese (Cobalt/Manganese = 10/1) to about 1000 moles of cobalt per mole of manganese (Cobalt/Manganese = 1000/1) and preferably it is in the range of from about 10 moles of cobalt per mole of manganese (Cobalt/Manganese = 10/1) to about 500 moles of cobalt per mole of manganese (Cobalt/Manganese = 500/1). More preferably, the molar ratio of cobalt to manganese is about 100 moles of cobalt per mole of manganese (Cobalt/Manganese = 100/1). The cobalt to manganese ratios set forth above for the preferred catalyst composition are equally applicable for other catalyst compositions contemplated in this invention. Thus for a catalyst composition comprising cobalt and a minor amount of molybdenum, the molar ratios between the cobalt and the molybdenum are the same as the ratios set forth above for the preferred catalyst of cobalt and manganese. The same molar ratios between the cobalt and the other metal are maintained when more than one other metal is used and, in that instance, the ratios are between the moles of cobalt and the sum of the moles of the other metals.

The amount of catalyst used in the reaction is a design choice which generally affects only the rate at which the oxidation reaction takes place. However, it is preferred that the molar ratio of the poly-alkyl poly-aromatic reactant to the cobalt portion of the catalyst present in the reaction mixture be within a range of from about 2 moles of reactant per mole of cobalt (Reactant/Cobalt = 2/1) to about 10,000 moles of reactant per mole of cobalt (Reactant/Cobalt = 10,000/1) and it is more preferred that the molar ratio of the reactant to the cobalt be within the range of from about 10 moles of reactant per mole of cobalt (Reactant/Cobalt = 10/1) to about 200 moles of reactant per mole of cobalt (Reactant/Cobalt = 200/1).

The purpose of the solvent is to dissolve the catalyst and the reactants and intermediates. The solvent comprises an organic aliphatic acid having from 2 to about 7 carbon atoms and is preferably a monocarboxylic acid. Acetic acid may be used in this process; however, propionic acid is preferred for the purposes of this invention. It has been found that the use of the preferred propionic acid, with the preferred catalyst, provides a process which gives higher selectivity to the desired poly-aromatic poly-carboxylic acids and produces less byproduct terephthalic acid than conventional processes using acetic acid and the conventional catalyst. The solvent chosen and the amount used are selected to ensure dissolution of the reactants, part of which may be in solution at the start of the reaction, and the catalyst during the reaction. The ratio of the solvent to the reactants on a weight basis will generally be within the range of from about 3 to 1 (Solvent/Reactants = 3/1 weight/weight) to about 50 to 1 (Solvent/Reactants = 50/1 weight/weight) and the ratio is preferably within the range of from about 5 to 1 (Solvent/Reactants = 5/1 weight/weight) to about 20 to 1 (Solvent/Reactant = 20/1 weight/weight).

In general the reaction will be conducted at a temperature between about 50°C. and about 240°C., preferably between about 100°C. and about 220°C., and most preferably between about 170°C. and 200°C. In any case, the temperature of the reaction will depend in part upon the catalyst, the reactant and the solvent used.

The reaction is usually conducted at pressures of about 100 kilopascals or greater, preferably at a pressure within a range of between about 100 kilopascals and about 14,000 kilopascals, and most preferably at a pressure within a range between about 700 kilopascals and about 1050 kilopascals. The reaction may be conducted at low temperature and atmospheric pressure, so long as the reactants are liquid; however, the rate of reaction will be slower than the rate if the temperature and pressure for the reaction were within the ranges set out above.

As indicated above, the reaction rate is sensitive to the reaction temperature and pressure as well as the concentration of the reactants and the catalyst. Reaction times of from about 2 hours to about 12 hours have been used, but one of ordinary skill can manipulate the variables that affect the rate of reaction to achieve longer or shorter reaction times.

The catalytic oxidation process of this invention is especially useful for the oxidation of tertiary alkyl groups. In addition, this invention can be used in the production of dicarboxy aromatics, which are of great interest to the high performance plastics industry and for use in liquid crystal polymers.

The practice of this invention is illustrated by the following specific examples. These examples are included for illustrative purposes only and are not intended to limit the scope of the invention.

EXAMPLE 1 - PRIOR ART

This Example illustrates the poor yield of desired product when only a cobalt metal salt catalyst is used. A 90 cubic centimeter glass reactor equipped with an efficient stirrer and gas sparger was charged with 0.0651 grams cobaltous acetate, 3.001 grams 4,4′-diisopropyl biphenyl and 40 cubic centimeters acetic acid. The reactor was pressurized to 620 kilopascals with compressed air and an air flow of 500 standard cubic centimeters per minute was maintained. The reactor was heated to 160°C. and held at that temperature for 8 hours. The reaction mixture was stirred continuously. A yield of 0% (zero %) of 4,4′-biphenyl dicarboxylic acid and a 26.6% conversion of the starting material was achieved.

## EXAMPLE 2 - PRIOR ART

This Example illustrates the poor yield of desired product when cobalt with bromine (Br-) is used as the catalyst. The reactor of Example 1 was charged with 0.066 grams cobaltous acetate, 0.029 cubic centimeters of 48% hydrobromic acid, 3.000 grams of 4,4′-diisopropyl biphenyl and 40 cubic centimeters of acetic acid. The reactor was pressurized to 620 kilopascals with compressed air and an air flow of 500 standard cubic centimeters per minute was maintained. The reactor was heated to 160°C. and held at that temperature for 8 hours. The reaction mixture was stirred continuously. A yield of 1% of 4,4′-biphenyl dicarboxylic acid and a 77.8% conversion of the starting materials was achieved.

## EXAMPLE 3 - PRIOR ART

This Example illustrates the poor yield of desired product when only a manganese metal salt catalyst is used. The reactor of Example 1 was charged with 0.0452 grams of manganese (II) acetate, 3.000 grams of 4,4′-diisopropyl biphenyl and 40 cubic centimeters of acetic acid. The reactor was pressurized to 620 kilopascals with compressed air and an air flow of 500 standard cubic centimeters per minute was maintained. The reactor was heated to 160°C. and held at that temperature for 8 hours. The reaction mixture was stirred continuously. A yield of 27.5% of 4,4′-biphenyl dicarboxylic acid and a 97.8% conversion of the starting materials was achieved.

## EXAMPLE 4 - PRIOR ART

This Example illustrates the yield that can be expected utilizing the typical process and catalyst of the prior art for the auto-oxidation of alkylated aromatics. The catalyst is a mixed metal catalyst, with a manganese to cobalt molecular ratio of 2 to 1 (manganese/cobalt = 2/1), with an added bromine (Br-) promoter. The reactor of Example 1 was charged with 2.560 grams of cobaltous acetate tetrahydrate, 5.337 grams of manganese (II) acetate, 1.050 grams of sodium bromide, 4.000 grams of 4,4′-diisopropyl biphenyl and 40 cubic centimeters of acetic acid. The reactor was pressurized to 1034 kilopascals with compressed air and an air flow of 500 standard cubic centimeters per minute was maintained. The reactor was heated to 180°C. and held at that temperature for 3 hours. The reaction mixture was stirred continuously. A yield of 57% of 4,4′-biphenyl dicarboxylic acid and a 72% conversion of the starting materials was achieved.

## EXAMPLE 5

This Example illustrates the yield that can be expected utilizing the typical process and catalyst of the prior art for the auto-oxidation of alkylated aromatics if the acetic acid is replaced with the propionic acid preferred in this invention. The catalyst is a prior art mixed metal catalyst, with a manganese to cobalt molecular ratio of 2 to 1 (manganese/cobalt = 2/1), with an added bromine (Br-) promoter. The reactor of Example 1 was charged with 2.560 grams of cobaltous acetate tetrahydrate, 5.335 grams of manganese (II) acetate, 1.050 grams of sodium bromide, 4.000 grams of 4,4′-diisopropyl biphenyl and 40 cubic centimeters of propionic acid. The reactor was pressurized to 1034 kilopascals with compressed air and an air flow of 500 standard cubic centimeters per minute was maintained. The reactor was heated to 180°C. and held at that temperature for 3 hours. The reaction mixture was stirred continuously. A yield of 77% of 4,4′-biphenyl dicarboxylic acid and a 95% conversion of the starting materials was achieved.

## EXAMPLE 6

This Example illustrates the improvement possible through the use of the process and catalyst of this invention in the auto-oxidation of 4,4′-diisopropyl biphenyl. Propionic acid was used as the solvent and the catalyst was a mixed metal catalyst having a cobalt to manganese molecular ratio of 100 to 1 (cobalt/manganese = 100/1). The reactor of Example 1 was charged with 0.065 grams of cobaltous acetate tetrahydrate, 0.00046 grams of manganese (II) acetate, 3.000 grams of 4,4′-diisopropyl biphenyl and 40 cubic centimeters of propionic acid. The reactor was pressurized to 793 kilopascals with compressed air and an air flow of 500 standard cubic centimeters per minute was maintained. The reactor was heated to 175°C. and held at that temperature for 4 hours. The reaction mixture was stirred continuously. A yield of 93% of 4,4′-biphenyl dicarboxylic acid and a 100% conversion of the starting materials was achieved.

## EXAMPLE 7

This Example illustrates the yield of the desired 4,4′-biphenyl dicarboxylic acid that can be achieved when acetic acid is used as the solvent in the process of this invention. The reactor of Example 1 was charged with 0.065 grams of cobaltous acetate tetrahydrate, 0.0045 grams of manganese (II) acetate, 3.0 grams of 4,4'-diisopropyl biphenyl and 38.4 grams of acetic acid. The reactor was pressurized to 965 kilopascals with compressed air and an air flow of 500 standard cubic centimeters per minute was maintained. The reactor was heated to 200°C. and held at that temperature for 2 hours. The reaction mixture was stirred continuously. A yield of 83% of 4,4'-biphenyl dicarboxylic acid and a conversion of the starting materials of greater than 99% was achieved.

EXAMPLE 8

This Example repeats Example 7 with propionic acid as the solvent to illustrate the improvement in yield that is possible when propionic acid is used rather than acetic acid. The reactor of Example 1 was charged with 0.065 grams of cobaltous acetate tetrahydrate, 0.0045 grams of manganese (II) acetate, 3.0 grams of 4,4'-diisopropyl biphenyl and 38.4 grams of propionic acid. The reactor was pressurized to 965 kilopascals with compressed air and an air flow of 500 standard cubic centimeters per minute was maintained. The reactor was heated to 200°C. and held at that temperature for 2 hours. The reaction mixture was stirred continuously. A yield of 91% of 4,4'-biphenyl dicarboxylic acid and a conversion of the starting materials of greater than 99% was achieved.

EXAMPLE 9

This example illustrates the general utility of the catalyst of this invention for the oxidation of a poly-alkyl polycyclic compound, 2,6-diisopropyl naphthalene. The reactor of Example 1 was charged with 0.065 grams of cobaltous acetate tetrahydrate, 0.0045 grams of manganese(II) acetate, 3.0 grams of 2,6-diisopropyl naphthalene and 38.3 grams of propionic acid. The reactor was pressurized to 1034 kilopascals with compressed air and an air flow of 500 standard cubic centimeters per minute was maintained. The reactor was heated to 160° C. and the temperature was maintained for 12 hours. The reaction mixture was stirred continuously. A yield of 62.9 % of 2,6-dicarboxynaphthalene and a 79.1 % conversion of the starting materials was achieved.

EXAMPLE 10

This example illustrates the feasibility of using the catalyst of this invention for the oxidation of alkyl-substituted aromatic compounds such as 4,4'-diisopropyl diphenyl ethers. The reactor of Example 1 was charged with 0.065 grams of cobaltous acetate tetrahydrate, 0.0046 grams of manganese(II) acetate, 3.0 grams of 4,4'-diisopropyl diphenyl ether and 38.6 grams of propionic acid. The reactor was pressurized to 1034 kilopascals with compressed air and an air flow of 500 standard cubic centimeters per minute was maintained. The reactor was heated to 160° C. and the temperature was maintained for 4 hours. The reaction mixture was stirred continuously. A yield of 12 % of 4,4'-dicarboxydiphenyl ether and a 52 % conversion of the starting materials was achieved.

It will be apparent from the examples that many other variations and modifications may be made in the processes and compositions described without departing from the concept of the invention. Accordingly, it should be understood that the description and examples are illustrative only and are not intended to limit the scope of the invention.

**Claims**

1. A process for the production of poly-aromatic acids comprising oxidizing the alkyl substituents of an alkyl substituted poly-aromatic compound with an oxygen containing gas in the presence of a catalytic amount of a halogen-free metal catalyst composition, said oxidation being carried out in a solvent, said solvent comprising an organic aliphatic carboxylic acid having from 2 to about 7 carbon atoms.

2. The process of claim 1 wherein said alkyl substituted poly-aromatic compound has at least two alkyl substituents.

3. The process of claim 2 wherein said alkyl substituted poly-aromatic compound has at least one alkyl substituent on each terminal aromatic ring.

4. The process of claim 3 wherein said alkyl substituent is an alkyl having from 1 to about 5 carbon atoms.

5. The process of claim 4 wherein said alkyl substituted poly-aromatic compound is a para-oriented poly-alkyl poly-aromatic compound.

6. The process of claim 5 wherein said alkyl substituted poly-aromatic compound is 4,4'-diisopropyl biphenyl.

7. The process of claim 1 wherein said poly-aromatic acid is a poly-carboxyl poly-aromatic acid.

8. The process of claim 7 wherein said poly-carboxyl poly-aromatic acid is 4,4'-biphenyl dicarboxylic acid.

9. The process of claim 1 wherein said halogen-free metal catalyst composition comprises a source of cobalt and a minor amount of a source of at least one other metal selected from the group consisting of cerium, gadolinium, manganese, molybdenum, tin, tungsten, vanadium and zirconium.

10. The process of claim 9 wherein the molecular ratio of said cobalt to said at least one other metal is within a range of from about 10 to 1 to about 1000 to 1.

11. The process of claim 10 wherein the molecular ratio of said cobalt to said at least one other metal is within a range of from about 10 to 1 to about 500 to 1.

12. The process of claim 11 wherein the molecular ratio of said cobalt to said at least one other metal is about 100 to 1.

13. The process of claim 9 wherein said halogen-free metal catalyst composition comprises a source of cobalt and a source of a minor amount of manganese.

14. The process of claim 13 wherein said halogen-free metal catalyst composition comprises a source of cobalt and a source of a minor amount of manganese wherein the molecular ratio of said cobalt to said manganese is within a range of from about 10 to 1 to about 1000 to 1.

15. The process of claim 14 wherein the molecular ratio of said cobalt to said manganese is within a range of from about 10 to 1 to about 500 to 1.

16. The process of claim 15 wherein the molecular ratio of said cobalt to said manganese is about 100 to 1.

17. The process of claim 13 wherein said source of cobalt is cobalt acetate.

18. The process of claim 13 wherein said source of manganese is manganese acetate.

19. The process of claim 1 wherein said solvent is acetic acid.

20. The process of claim 1 wherein said solvent is propionic acid.

21. The process of claim 5 wherein said alkyl substituted polyaromatic compound is 4,4'-di-secondary butyl biphenyl.

22. A process for the production of poly-carboxyl poly-aromatic acids comprising oxidizing at least two alkyl substituents of an alkyl substituted aromatic compound having at least two alkyl substituents with an oxygen containing gas in the presence of a catalytic amount of a halogen-free metal catalyst composition comprising a cobalt salt and a minor amount of a manganese salt, said oxidation being carried out in a solvent, said solvent comprising an organic aliphatic carboxylic acid having from 2 to about 7 carbon atoms.

23. The process of claim 22 wherein said halogen-free metal catalyst composition comprises a cobalt salt and a minor amount of a manganese salt wherein the molecular ratio of said cobalt to said manganese is within a range of from about 10 to 1 to about 1000 to 1.

24. The process of claim 23 wherein the molecular ratio of said cobalt to said manganese is within a range of from about 10 to 1 to about 500 to 1.

25. The process of claim 24 wherein the molecular ratio of said cobalt to said manganese is about 100 to 1.

26. The process of claim 22 wherein said solvent is acetic acid.

27. The process of claim 22 wherein said solvent is propionic acid.

28. The process of claim 22 wherein said alkyl substituents have from 1 to about 5 carbon atoms.

29. A catalyst composition comprising a cobalt salt and a minor amount of a manganese salt, said cobalt and said manganese being present in a molecular ratio of cobalt to manganese within a range of from about 10 to 1 to about 1000 to 1, all dissolved in a solvent therefore, said solvent comprising an organic aliphatic carboxylic acid having from 2 to about 7 carbon atoms.

30. The catalyst composition of claim 29 wherein said molecular ratio of cobalt to manganese is within a range of from about 10 to 1 to about 500 to 1.

31. The catalyst composition of claim 30 wherein said molecular ratio of cobalt to manganese is about 100 to 1.

32. The catalyst composition of claim 29 wherein said solvent is acetic acid.

33. The catalyst composition of claim 29 wherein said solvent is propionic acid.

34. A process for the production of aromatic acids

**EP 0 475 926 A2**

comprising oxidizing at least one alkyl group of an alkyl substituted poly-alkyl poly-aromatic compound with an oxygen containing gas in the presence of a catalytic amount of a halogen-free metal catalyst composition comprising a cobalt salt and a minor amount of a manganese salt, said oxidation being carried out in a solvent, said solvent comprising an organic aliphatic carboxylic acid having from 2 to about 7 carbon atoms.

35. The process of claim 34 wherein said solvent is propionic acid.

36. The process of claim 34 wherein said halogen-free metal catalyst composition comprises a cobalt salt and a minor amount of a manganese salt wherein the molecular ratio of said cobalt to said manganese is within a range of from about 10 to 1 to about 500 to 1.

37. A process for the production of 4,4'-biphenyl dicarboxylic acid comprising oxidizing 4,4'-diisopropyl biphenyl with an oxygen containing gas in the presence of a catalytic amount of a halogen-free metal catalyst composition, said catalyst composition comprising a cobalt salt and a minor amount of a manganese salt wherein the molar ratio of said cobalt to said manganese is within a range of from about 10 to 1 to about 500 to 1, said oxidation being carried out in propionic acid.

38. A process for the production of poly-aromatic acids comprising oxidizing the alkyl substituents of an alkyl substituted poly-aromatic compound with an oxygen containing gas in the presence of a catalytic amount of a metal catalyst composition, said catalyst composition comprising a cobalt salt and a minor amount of a manganese salt wherein the molar ratio of said cobalt to said manganese is within a range of from about 10 to 1 to about 500 to 1, said oxidation being carried out in a solvent, said solvent comprising an organic aliphatic carboxylic acid having from 2 to about 7 carbon atoms.